Europäisches Patentamt

European Patent Office

Office européen des brevets

(11) Veröffentlichungsnummer: **0 203 345 B1**

# EUROPÄISCHE PATENTSCHRIFT

(45) Veröffentlichungstag der Patentschrift: **20.06.90**

(51) Int. Cl.⁵: **A 61 F 7/00**

(21) Anmeldenummer: **86105345.2**

(22) Anmeldetag: **17.04.86**

(54) Gerät zur Erzeugung eines kalten Behandlungsgases.

(30) Priorität: **22.05.85 DE 8514989 u**

(43) Veröffentlichungstag der Anmeldung:
**03.12.86 Patentblatt 86/49**

(45) Bekanntmachung des Hinweises auf die
Patenterteilung:
**20.06.90 Patentblatt 90/25**

(84) Benannte Vertragsstaaten:
**AT BE CH FR GB IT LI NL SE**

(56) Entgegenhaltungen:
**AT-B- 376 032
DE-A-3 242 881
DE-A-3 305 434**

(73) Patentinhaber: **MESSER GRIESHEIM GMBH
Hanauer Landstrasse 330
D-6000 Frankfurt/Main (DE)**

(72) Erfinder: **Fieseler, Heinrich
Kantstrasse 2
D-4047 Dormagen 5 (DE)**
Erfinder: **Thoma, Klemens
Am Kleckers 22
D-4150 Krefeld-Hüls 29 (DE)**

Courier Press, Leamington Spa, England.

**Beschreibung**

Die Erfindung betrifft ein Gerät zur Erzeugung einft ein Gerät zur Erzeugung eines kalten Behandlungsgases aus Stickstoff und Luft für die Kryotherapie rheumatischer Erkrankungen nach dem Oberbegriff des Anspruches 1.

Für die Kryotherapie rheumatischer Erkrankungen werden vielfach Geräte benutzt, bei denen das kalte Behandlungsgas durch Verdampfung von flüssigem Stickstoff gewonnen wird. Für die Wärmeübertragung dient Luft, welche mit dem verdampften Stickstoff vermischt wird. Derartige Geräte, wie sie beispielsweise aus den deutschen Offenlegungsschriften 32 42 881 und 33 05 434 bekannt sind, sind für Krankenhäuser oder Therapiezentren geeignet, wo täglich eine größere Anzahl von Behandlungen vorgenommen wird.

Für diese Großgeräte ist ein ziemlich hoher Überwachungs und Sicherheitsaufwand erforderlich. So muß der Sauerstoffgehalt im Behandlungsraum überwacht werden, der bei mangelnder Entlüftung oder einer fehlerhaften Totalverdampfung des flüssigen Stickstoffs auf einen unzulässigen Wert absinken könnte. Es muß gewährleistet sein, daß in dem Speicherbehälter für flüssigen Stickstoff keine Luft kondensieren kann. Hierdurch könnten sich Sauerstoffanreicherungen im flüssigen Stickstoff bilden, aus denen bei der nachfolgenden Verdampfung explosive Gasgemische entstehen könnten. Außerdem sind wirksame Einrichtungen zur Trocknung der Luft oder des Behandlungsgases erforderlich, da ausgefrorene Feuchtigkeit nicht in Kontakt mit den zu behandelnden Körperpartien kommen darf. Für normale Arztpraxen, in denen nur gelegentlich eine Behandlung durchgeführt wird, sind diese Geräte daher weniger geeignet.

Der Erfindung liegt daher die Aufgabe zugrunde, ein Gerät für die Kryotherapie zu schaffen, welches für normale Arztpraxen geeignet ist und nur einen geringen Aufwand für Sicherheit und Überwachung erfordert.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Die Erfindung beruht demnach auf dem Gedanken, den Speicher für flüssigen Stickstoff so klein zu machen, daß mit der gespeicherten Stickstoffmenge tatsächlich nur eine oder höchstens einige wenige Behandlungen durchgeführt werden können. Er muß praktisch für jede Behandlung aus einem üblichen größeren Behälter zur Aufbewahrung von flüssigem Stickstoff aufgefüllt werden. Für die Behandlung wird der gespeicherte Stickstoff entweder völlig verbraucht oder doch zum größten Teil. Der nicht verbrauchte flüssige Stickstoff kann nachfolgend durch den natürlichen Wärmeeinfall ebenfalls verdampfen. Wegen der geringen Menge an verdampftem Stickstoff, die hier überhaupt entstehen kann, kann auf eine Sauerstoffüberwachung verzichtet werden, da der Sauerstoffgehalt auf keinen Fall unter ein zulässiges Maß absinken kann. Da der Behälterinhalt nach jeder Behandlung total verdampft, besteht auch keine Gefahr einer unzulässigen Sauerstoffanreicherung. Diese Gefahr wird noch dadurch verringert, daß die zur Verdampfung des flüssigen Stickstoffes dienende Luft nicht direkt mit dem flüssigen Stickstoff in Berührung kommt.

Entsprechend den geringen Stickstoffmengen werden auch nur geringe Mengen an Luft benötigt. Die Luft kann daher durch ein kleines Gebläse erzeugt werden, welches mit dem Behälter für den flüssigen Stickstoff vorzugsweise fest auf einem Wagen installiert ist, so daß das Gerät leicht an den Ort des Bedarfs gebracht und wieder weggeschafft werden kann. Wegen der geringen Mengen an Behandlungsgas ist auch die Trocknung des Luftstroms unproblematisch. Es können hierfür Adsorptionstrockner oder Molekularsiebe verwendet werden. Die Luftfeuchtigkeit kann auch in einem Wärmeaustauscher auskondensiert werden. Ein Adsorptionstrockner kann beispielsweise in der nachfolgenden Ruhepause durch den verdampfenden restlichen Stickstoff durchströmt und regeneriert werden.

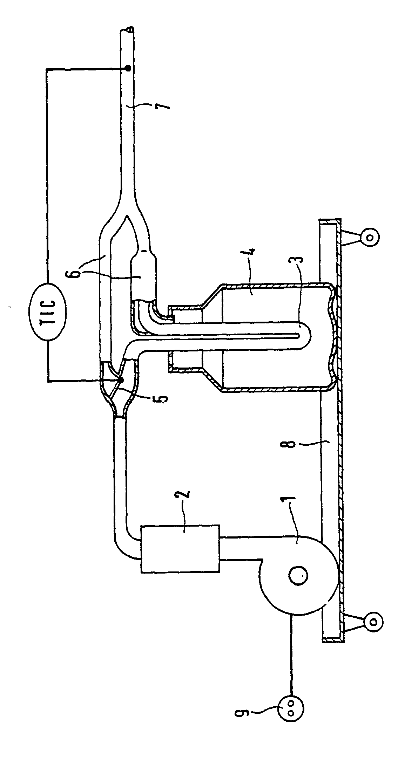

Die Zeichnung veranschaulicht ein Ausführungsbeispiel der Erfindung in schematischer Form.

Das in der Zeichnung dargestellte Gerät besteht aus zwei wesentlichen Komponenten, nämlich dem Gebläse 1 mit dem nachgeschalteten Trockner 2 und der wärmeübertragenden Einrichtung 3 im Behälter 4 für flüssigen Stickstoff. Beide Komponenten sind durch eine Mischstrecke 6 miteinander verbunden. Die Mischstrecke 6 besitzt an ihrem Eintritt eine verstellbare Klappe 5, durch welche der vom Gebläse 1 kommende Strom verdichteter Luft in zwei Teilströme aufgeteilt wird. Der eine Teilstrom durchströmt die wärmeübertragende Einrichtung 3, wodurch flüssiger Stickstoff verdampft wird. Der verdampfte Stickstoff vermischt sich mit diesem Teilstrom in der Mischstrecke 6, in welcher auch als zweiter Teilstrom nicht abgekühlte Luft wieder zugemischt wird. Das fertige kalte Behandlungsgas aus Stickstoff und Luft steht dann in der Austrittsleitung 7 zur Verfügung. Die Temperatur des Behandlungsgases wird gemessen und geregelt. Die Regelung erfolgt durch Betätigen der verstellbaren Klappe 5, wodurch der Anteil der durch die wärmeübertragende Einrichtung 3 strömenden verdichteten Luft vergrößert, bzw. verkleinert werden kann. Entsprechend verändert sich die Menge des verdampften Stickstoffs. Die gesamte Einrichtung ist auf einem Wagen 8 installiert. Sie benötigt lediglich einen einfachen elektrischen Anschluß 9, um in Betrieb gesetzt zu werden.

Vor der Inbetriebnahme wird der Behälter 4 mit flüssigem Stickstoff aufgefüllt. Nach dem Ende der Behandlung befindet sich im Behälter 4 gewöhnlich noch ein Rest von flüssigem Stickstoff, der allmählich durch Wärmeeinfall verdampft. Dieser verdampfte Stickstoff kann dazu ausgenutzt werden, den Trockner 2 zu regenerieren oder dort auskondensierte Feuchtigkeit zu entfernen.

An sich ist die typische Betriebsweise des erfindungsgemäßen Gerätes, die darin besteht, vor jeder Behandlung den Behälter 4 mit flüssigem Stickstoff aufzufüllen, nicht wirtschaftlich, da der Behälter 4 und die Mischstrecke 6 mit der Austrittsleitung 7 jedesmal auf tiefe Temperaturen abgekühlt werden müssen. Die Verluste, die hierdurch entstehen, sind jedoch klein, da die abzukühlenden Massen klein sind. Demgegenüber besteht der Vorteil, daß weitgehend auf Überwachungs- und Kontrolleinrichtungen verzichtet werden kann. Durch den geringen Inhalt des Behälters 4 besteht keine Gefahr einer unzulässig hohen Sauerstoffverdrängung in der Raumluft bei Behandlungen. Ein Sauerstoffmeßgerät kann somit entfallen. Da das Rohrleitungssystem der wärmeübertragenden Einrichtung 3 geschlossen ist, kann auch keine Sauerstoffanreicherung im flüssigen Stickstoff erfolgen. Es ist außerdem möglich, die Fördermenge des Luftstromes durch das Gebläse 1 zu variieren, um so den unterschiedlichsten Behandlungsanforderungen gerecht zu werden. Sofern bei der Kälteapplikation nur Einzelbehandlungen durchgeführt werden und das Behandlungsgerät jeweils neu abgekühlt werden muß, wirken die Leitungen als Kondensationsfläche, an denen sich Feuchtigkeit niederschlagen kann. Wenn aus medizinischer Sicht die Applikation eines trockenen Kaltgasstromes unumgänglich ist, ist die Vorschaltung eines Trockners, wie z.B. Molekularsieb, möglich.

Neben der Kryotherapie im medizinischen Bereich sind auch weitere Anwendungen in technischen Disziplinen denkbar, bei denen das Gerät auch über eine längere Betriebszeit eingesetzt wird. Auskondensierte Luftfeuchtigkeit im Kaltgasstrom würde in diesem Fall die Anwendung nicht behindern. Derartige Anwendungen sind z.B. der Einsatz bei Gefrierversuchen, die Kühlung von Prüf- oder Klimakammern, in denen eine kalte Gastemperatur, beispielsweise für die Prüfung von Bauteilen eingehalten werden soll, oder die Kühlung von Oberflächen.

Alternativ zur Temperaturregelung mittels Zuführung warmer Luft über eine verstellbare Klappe kann der Kaltgasstrom vor dem Austritt aus dem Schlauch über ein Heizregister geführt und elektrisch auf die gewünschte Austrittstemperatur geheizt werden.

## Patentansprüche

1. Gerät zur Erzeugung eines kalten Behandlungsgases aus Stickstoff und Luft für die Kryotherapie rheumatischer Erkrankungen, mit dem flüssiger Stickstoff verdampft und mit der unter erhöhtem Druck stehenden trockenen Luft im gewünschten Verhältnis gemischt wird, dadurch gekennzeichnet, daß ein Behälter (4) für die Speicherung des flüssigen Stickstoffs vorgesehen ist, der eine Aufnahmekapazität zur Durchführung von wenigstens einer und höchstens einigen wenigen Behandlungen besitzt, daß in dem Behälter wärmeübertragende Einrichtungen (3) zur Durchleitung eines Teils der unter erhöhtem Druck stehenden Luft angeordnet sind, und daß ferner zur Schaffung der unter erhöhtem Druck stehenden trockenen Luft ein mit einem Trockner (2) versehenes Gebläse (1) vorgesehen ist, dessen Austrittsleitung aufgeteilt ist in einen Anschluß an eine an den Behälter (4) angeschlossene Mischstrecke (6) und in einen Anschluß an die wärmeübertragenden Einrichtungen (3), wobei die Mischstrecke (6) zur Vermischung von verdampftem Stickstoff aus dem Behälter (4), von abgekühlter Luft aus den wärmeübertragenden Einrichtungen (3) und von nichtabgekühlter Luft aus dem Anschluß der Gebläseaustrittsleitung an die Mischstrecke dient.

2. Gerät nach Anspruch 1, gekennzeichnet durch eine von der Temperatur des Behandlungsgases geregelte Verstelleinrichtung zur Dosierung der Luftmengen für die Mischstrecke und für die wärmeübertragende Einrichtung.

3. Gerät nach Anspruch 1 oder 2, dadurch gekennzeichnet, daß Gebläse und Behälter auf einem Wagen (8) fest installiert sind.

4. Gerät nach Anspruch 2 oder 3, dadurch gekennzeichnet, daß die Verstelleinrichtung eine Klappe (5) ist.

5. Gerät nach einem der Ansprüche 1 bis 4, dadurch gekennzeichnet, daß die wärmeübertragende Einrichtung eine Rohrleitung ist.

6. Gerät nach Anspruch 1, dadurch gekennzeichnet, daß im Anschluß an die Mischstrecke ein elektrisches Heizregister angeordnet ist, mit dem die Einstellung der Temperatur des kalten Behandlungsgases vorgenommen wird.

## Revendications

1. Dispositif pour la production d'un gaz froid pour traitement médical, formé d'azote et d'air, pour la cryothérapie de maladies rhumatismales, dans lequel l'azote liquide est vaporisé et mélangé en proportion désirée avec l'air sec présent sous pression, caractérisé en ce qu'il est prévu un réservoir (4) pour le stockage de l'azote liquide, ce réservoir ayant une capacité de réception pour l'exécution d'au moins un traitement et au plus quelques petits traitements, de telle sorte que dans le réservoir sont disposés des dispositifs (3) de transmission de chaleur pour être traversés par une partie de l'air mis sous haute pression, et que, de plus, pour obtenir l'air sec sous haute pression, il est prévu un ventilateur (1) muni d'un sécheur (2), dont la conduite de sortie est divisée en un raccordement avec une conduite (6) de mélange reliée au réservoir (4), et un autre raccordement avec les dispositifs (3) transmettant la chaleur, grâce à quoi la conduite (6) de mélange sert au mélange de l'azote vaporisé en provenance du réservoir (4), de l'air réfrigéré en provenance des dispositifs (3) transmettant la chaleur, et de l'air non réfrigéré en provenance du raccordement de la conduite de sortie du ventilateur avec la conduite de mélange.

2. Dispositif selon la revendication 1, caractérisé par un dispositif de réglage réglé par la température du gaz de traitement, pour le dosage des quantités d'air pour la conduite de mélange et pour le dispositif transmettant la chaleur.

3. Dispositif selon les revendications 1 ou 2, caractérisé en ce que le ventilateur et le réservoir sont installés de façon fixe sur un chariot (8).

4. Dispositif selon les revendications 2 ou 3, caractérisé en ce que le dispositif de réglage est un clapet (5).

5. Dispositif selon l'une quelconque des revendications 1 à 4, caractérisé en ce que le dispositif transmettant la chaleur est une conduite tubulaire.

6. Dispositif selon la revendication 1, caractérisé en ce que, au raccordement de la conduite de mélange, un enregistreur de température est disposé, par lequel le réglage de la température du gaz froid de traitement est choisi.

**Claims**

1. Apparatus for producing a cold therapeutic gas from nitrogen and air for the cryotherapy of rheumatic illnesses, with which liquid nitrogen is vaporized and mixed in the desired proportion with dry air subjected to high pressure, characterized in that a container (4) is provided for storing the liquid nitrogen, which possesses a capacity for executing at least one and at most a few treatments, in that heat-transferring devices (3) for transferring a part of the air subjected to high pressure are arranged in the container, and in that, further, for the purpose of producing the dry air subjected to high pressure a blower (1), provided with a drier (2), is provided of which the outlet line is subdivided into a connection to the mixing section (6), which is connected to the container (4), and into a connection to the heat-transferring devices (3), the mixing section (6) serving to mix vaporized nitrogen from the container (4), cooled air from the heat-transferring devices (3) and uncooled air from the connection of the blower outlet line to the mixing section.

2. Apparatus according to Claim 1, characterized by an adjusting element, regulated by the therapeutic gas, for dosing the air volumes for the mixing section and for the heat-transferring device.

3. Apparatus according to Claim 1 or 2, characterized in that the blower and the container are permanently installed on a carriage (8).

4. Apparatus according to Claim 2 or 3, characterized in that the adjusting element is a flap (5).

5. Apparatus according to one of Claims 1 to 4, characterized in that the heat-transferring device is a pipeline.

6. Device according to Claim 1, characterized in that an adjustable electric heater is arranged in the connection to the mixing section, with which the setting of the temperature of the cold therapeutic gas is undertaken.